# EUROPÄISCHE PATENTSCHRIFT

(11) **EP 2 027 056 B1**
(45) Veröffentlichungstag und Bekanntmachung des Hinweises auf die Patenterteilung: **19.07.2017**
(21) Anmeldenummer: 07725342.5
(22) Anmeldetag: 18.05.2007
(51) Int. Cl.: B67C 7/00, B65G 47/84, A61L 2/06

(54) **VERFAHREN SOWIE VORRICHTUNG ZUM STERILISIEREN VON FLASCHEN ODER DERGLEICHEN BEHÄLTER**
METHOD AND DEVICE FOR STERILIZING BOTTLES OR SIMILAR CONTAINERS
PROCÉDÉ ET DISPOSITIF DE STÉRILISATION DE BOUTEILLES OU DE CONTENANTS SIMILAIRES

(30) Priorität: 20.05.2006 DE 102006023764
(43) Veröffentlichungstag der Anmeldung: 25.02.2009
(73) Patentinhaber: KHS GmbH, 44143 Dortmund (DE)
(72) Erfinder: EULER, Tobias, 55545 Bad Kreuznach (DE); METTE, Detlef, 55583 Bad Münster (DE); BECKMANN, Jörg, 21680 Stade (DE)
(86) Internationale Anmeldenummer: PCT/EP2007/004434
(87) Internationale Veröffentlichungsnummer: WO 2007/134803

(56) Entgegenhaltungen:
- EP-A- 0 334 288
- WO-A-01/29528
- DE-A1- 10 355 183
- DE-U1- 8 700 988
- FR-A- 2 576 003
- US-A1- 2004 208 781

## Beschreibung

Die Erfindung bezieht sich auf ein Verfahren gemäß Oberbegriff Patentanspruch 1 sowie auf eine Vorrichtung gemäß Oberbegriff Patentanspruch 20.

Die Sterilisation von Flaschen oder dergleichen Behälter durch die Behandlung mit einem erhitzten Sterilisationsmedium in Form eines Wasserstoffperoxyd enthaltenden Mediums (z. B. Aerosol), und durch anschließende Behandlung mit Heißluft ist bekannt. Die beiden Behandlungsschritte werden jeweils auf einer Behandlungsstrecke konstanter Länge durchgeführt, und zwar an Behandlungsstationen, die am Umfang eines um eine vertikale Achse umlaufend angetriebenen Rotors gebildet sind. Die sterilisierten Behälter werden dann in der Regel an eine Füllmaschine zum sterilen bzw. aseptischen Abfüllen eines flüssigen Füllgutes weitergeleitet.

Zur Überprüfung einer erfolgreichen Durchführung der Behandlungsschritte mit dem erhitzten Sterilisationsmedium und mit Heißluft wird am Ende jeder Behandlungsstrecke die Temperatur der Behälter beispielsweise mittels eines berührungslosen Temperatursensors ermittelt. Weicht die Behältertemperatur übermäßig stark von einem Temperatursollwert ab, so erfolgt ein Ausschleusen des jeweiligen Behälters und/oder das Absetzen eines Alarms und/oder das Abschalten der Sterilisationsanlage und des anschließenden Füllers.

Der Temperaturüberwachung liegt dabei die Erkenntnis zugrunde, dass nur eine ausreichend lange und damit erfolgreiche Behandlung der Behälter mit dem erhitzten Behandlungsmedium zu einer bestimmten Behältertemperatur führt. Nachteilig hierbei ist aber, dass die Behandlungswirkung und damit auch die Temperatur der Behälter von verschiedenen Parametern bzw. Einflussgrößen abhängig sind, so u.a. von der Art und Größe der Behälter, von der Maschinenleistung, d.h. von der Anzahl der behandelten Behälter je Zeiteinheit, sowie auch von dem Abkühlprozess, den die Behälter während der Behandlung unterliegen und der seinerseits wiederum von verschiedenen, auch zufälligen Einflussgrößen abhängig ist, nämlich u.a. von der Umgebungstemperatur, Luftströmung, Luftfeuchte usw. Aus diesen Gründen ist es bei bekannten Anlagen notwendig, den Toleranzbereich für Abweichungen der jeweils am Ende einer Behandlungsstrecke gemessenen Behältertemperatur von dem Temperatursollwert relativ breit zu wählen, beispielsweise in der Größenordnung von +/- 20°C.

Aus der Druckschrift DE 103 55 183 A1 ist dabei ein Verfahren zum Handhaben von Gegenständen bekannt geworden, wobei die Gefäße an einer Einlaufstation an einen Umlaufförderer übergeben, vom Umlaufförderer zunächst an einer Auslaufstation und wieder an der Einlaufstation vorbei zur Auslaufstation gefördert und frühestens beim zweiten Erreichen der Auslaufstation aus dem Umlaufförderer entfernt werden, wobei eine Strecke in Förderrichtung zwischen der Einlaufstation und der Auslaufstation mehrfach durchlaufen wird. Die Sterilisation von Behältern mittels eines erhitzten Behandlungsmediums ist beispielsweise bekannt aus den Druckschriften US 2004/0208781 A1 und EP 0 334 288 A1.

Aufgabe der Erfindung ist es, ein Verfahren aufzuzeigen, mit welchem eine verbesserte Überprüfung der Behandlung von Flaschen oder dergleichen Behältern mit einem Behandlungsmedium erreichbar ist. Zur Lösung dieser Aufgabe ist ein Verfahren entsprechend dem Patentanspruch 1 ausgebildet. Eine Vorrichtung zum Durchführen des Verfahrens ist Gegenstand des Patentanspruchs 20.

Bei einer generellen Ausführungsform des erfindungsgemäßen Verfahrens wird in völliger Abkehr von den bisherigen Vorstellungen die jeweilige Schaltposition, an der die Behandlung eingeleitet wird, in Abhängigkeit von der am Ende der Behandlungsstrecke oder unmittelbar nach der Behandlungsstrecke gemessenen Temperatur der Behälter im Sinne einer Verlängerung und/oder Verkürzung der Behandlungsstrecke variiert, und zwar unter Berücksichtigung einer Solltemperatur, die beispielsweise für unterschiedliche Behälterarten und/oder-größen in einem Speicher einer elektronischen Mess- und Steuereinrichtung abgelegt ist. Dies ist auf besonders einfache Weise durch entsprechende Ansteuerung der Behandlungsstationen an dem jeweiligen Transportelement oder Transporteur möglich.

Bei einer weiteren generellen Ausführungsform der Erfindung erfolgt ebenfalls in Abhängigkeit von der am Ende der Behandlungsstrecke bzw. unmittelbar nach der Behandlungsstrecke gemessenen Temperatur der Behälter eine Verlagerung der Endposition, an der die jeweilige Behandlung beendet wird, und zwar wiederum im Sinne einer Vergrößerung oder Verkleinerung der Behandlungsstrecken und vorzugsweise unter Berücksichtigung einer Solltemperatur.

Weiterhin besteht die Möglichkeit, die Änderung der Behandlungsstrecke gesteuert durch Betriebsparameter, wie Anzahl der je Zeiteinheit behandelten Behälter, Umgebungstemperatur, Luftfeuchtigkeit usw. zu steuern bzw. zu regeln und dann am Ende jeder Behandlungsstrecke allein für Überwachungszwecke die Behältertemperatur zu ermitteln.

Weiterhin ist es auch möglich, die Endposition, an der die Behandlung beendet wird bestimmten Betriebsparametern entsprechend, beispielsweise entsprechend der Behälterart und/oder -größe einzustellen, wobei dann die Schaltposition temperaturgesteuert, d.h. durch Messung der Temperatur der Behälter und vorzugsweise durch Vergleich mit einer Solltemperatur im Sinne einer Verlängerung oder Verkürzung der Behandlungsstrecke variiert wird, sodass die gemessene Temperatur der Behälter (Istwert) möglichst exakt der Solltemperatur (Sollwert) entspricht.

Die verschiedenen Methoden der Steuerung und/oder Überwachung der Behandlung können auch kombiniert werden.

Das erfindungsgemäße Verfahren hat den Vorteil, dass die Behandlung der Behälter unabhängig von äußeren Einflussgrößen, insbesondere unabhängig von der Maschinenleistung (Anzahl der behandelten Behälter je Zeiteinheit) sowie auch unabhängig von einem durch Umwelteinflüsse bestimmten Abkühlprozess Behälter ist, und dass eine Einflussnahme externer Faktoren, insbesondere auch von sich zufällig ergebenden, nicht steuerbaren Faktoren auf die Behandlung und/oder deren Überwachung, wie beispielsweise Umgebungstemperatur, Luftfeuchtigkeit, Luftströmungen usw. weitestgehend ausgeschlossen ist.

Weiterbildungen der Erfindung sind Gegenstand der Unteransprüche. Die Erfindung wird im Folgenden anhand der Figuren an einem Ausführungsbeispiel näher erläutert. Es zeigen:
- Fig. 1: in sehr vereinfachter Darstellung und in Draufsicht eine Anlage zum Sterilisieren von Flaschen oder dergleichen Behälter, insbesondere für ein kaltaseptisches Abfüllen eines flüssigen Füllgutes, beispielsweise von Fruchtsäften oder Milchprodukten, bestehend aus einem Sterilisator sowie aus zwei an den Sterilisator anschließenden Aktivatoren;

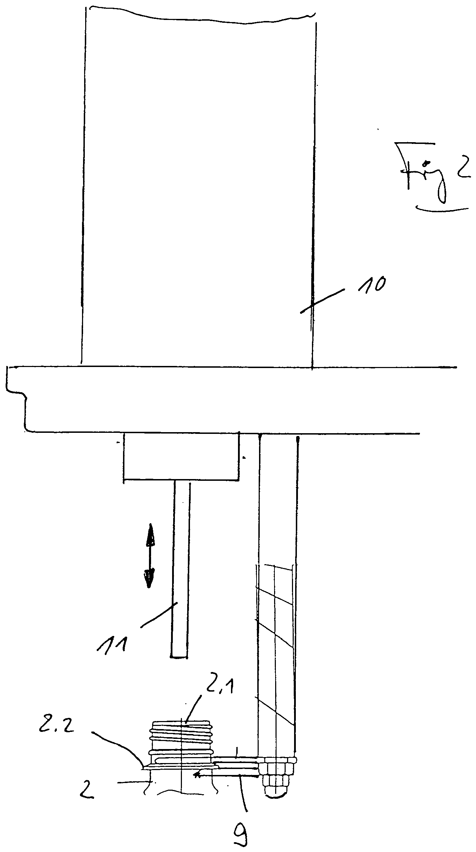

- Fig. 2: in vereinfachter Darstellung einen der Behandlungsköpfe des Sterilisators bzw. der Aktivatoren.

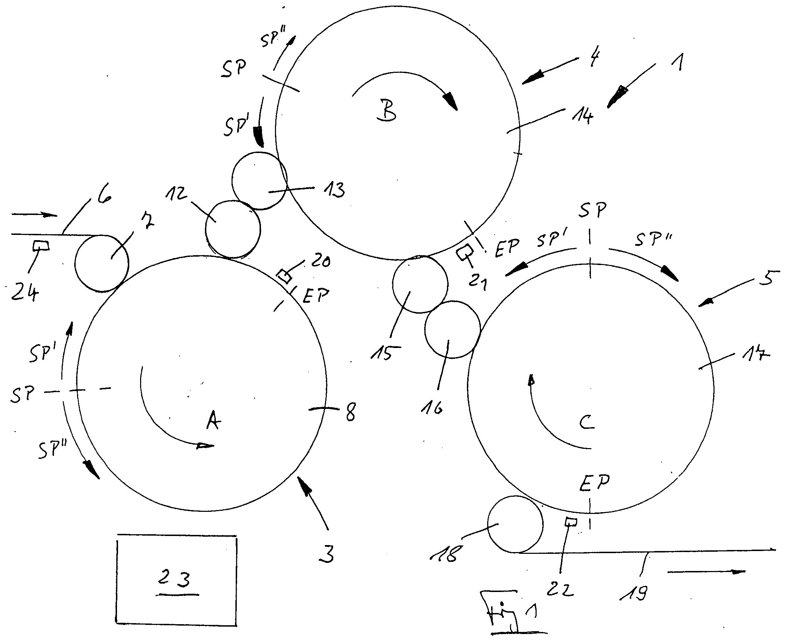

Die in den Figuren allgemein mit 1 bezeichnete Anlage dient zum Sterilisieren von Flaschen 2 durch Einbringen eines erhitzten Sterilisationsmediums in Form eines erhitzten Aerosols bestehend aus Luft und Wasserstoffperoxyd (H₂O₂) in die Flaschen sowie durch anschließendes Behandeln mit Heißluft zum Aktivieren des Wasserstoffperoxyds sowie zum Ausblasen des Sterilisationsmediums. Hierfür umfasst die Anlage einen in der Figur 1 allgemein mit 3 bezeichneten Sterilisator, in dem das Beaufschlagen der Flaschen 2 mit dem Sterilisationsmedium erfolgt, sowie zwei in der Figur 1 mit 4 bzw. 5 bezeichneten Aktivatoren, in denen das Behandeln der Flaschen 2 mit Heißluft zum Aktivieren sowie zum Ausblasen bzw. entfernen des Sterilisationsmediums erfolgt.

Der Sterilisator 3, dem die Flaschen 2 aufrecht stehend, d.h. mit ihrer Flaschenachse in vertikaler Richtung orientiert über einen Transporteur 6 und einen den Flascheneinlauf bildenden Transportstern 7 zugeführt werden, besteht im Wesentlichen aus einem um eine vertikale Maschinenachse umlaufend antreibbaren Rotor 8, der an seinem Umfang eine Vielzahl von Behandlungsstationen bildet. Die jeweils einzeln nacheinander von dem Einlaufstern 7 an einem Behälterträger 9 weitergeleiteten Flaschen 2 sind an diesen mit einem im Bereich der Flaschenmündung 2.1 versehenen Flansch 2.2 hängend gehalten.

Jeder Behandlungskopf 10 besitzt u.a. ein Behandlungsrohr 11, welches mit seinem offenen Ende durch die Flaschenmündung 2.1 in die Flasche 2 einführbar ist und aus welchem während der Behandlung das erhitzte Sterilisationsmedium in den Innenraum der betreffenden Flasche 2 eingeleitet wird. Die mit dem Sterilisationsmedium beaufschlagten Flaschen 2 gelangen dann über zwei umlaufend angetriebene Transportsterne 11 und 12, von denen der Transportstern 11 den Auslaufstern des Sterilisators 3 und der Transportstern 12 den Einlaufstern des ersten Aktivators 4 bilden, an jeweils eine Behandlungsstation dieses Aktivators 4, der wiederum im Wesentlichen von einem um eine vertikale Achse umlaufend antreibbaren Rotor 13 gebildet ist. Die am Umfang dieses Rotors 13 vorgesehenen Behandlungsstationen sind in gleicher Weise wie die vorbeschriebenen Behandlungsstationen des Sterilisators 3 ausgebildet, d.h. auch die Behandlungsstationen des Aktivators 4 bestehen jeweils aus einem Behälter- oder Flaschenträger 9 und einem Behandlungskopf 10, wobei allerdings im Aktivator 4 über das jeweilige Behandlungsrohr 11 Heißluft in die Flasche 2 eingeblasen wird, und zwar in zum Aktivieren des Sterilisationsmediums, d.h. des Wasserstoffperoxyds (H₂O₂) sowie auch zum Ausblasen bzw. Trockenblasen der Flaschen 2. Von dem Aktivator 4 bzw. den dortigen, am Rotor 14 gebildeten Behandlungsstationen gelangen die Flaschen 2 dann über Transportsterne 15 und 16, von denen der Transportstern 15 den Auslaufstern des Aktivators 4 und der Transportstern 16 den Einlaufstern des Aktivators 5 bilden, jeweils an eine Behandlungsstation des Aktivators 5. Dieser besteht ebenfalls im Wesentlichen aus einem um eine vertikale Achse umlaufend antreibbaren Rotor 17, der an seinem Umfang wiederum eine Vielzahl von Behandlungsstationen zur Aufnahme jeweils einer Flasche 2 und zum Einbringen von Heißluft in diese Flaschen 2 aufweist, und zwar ebenfalls zur Aktivierung des Sterilisationsmediums bzw. Aufrechterhaltung dieser Aktivierung und/oder zum endgültigen Ausblasen bzw. Trockenblasen der Flaschen 2. Die behandelten Flaschen 2 gelangen dann über einen Auslaufstern 18 an einen Transporteur 19, über den sie einer Füllmaschine zugeführt werden.

Die Drehrichtung der Rotoren 8, 14, 17 ist in der Figur 1 jeweils mit den Pfeilen A, B und C angegeben.

Die Behandlungsstationen an den Aktivatoren 4 und 5 bzw. an den dortigen Rotoren 14 und 17 sind in der gleichen Weise wie die Behandlungsstationen des Sterilisators 3 am Rotor 8 ausgebildet, d.h. die Behandlungsstationen bestehen jeweils aus einem Behälterträger 9 und aus einem Behandlungskopf 10, allerdings mit dem Unterschied, dass bei den Aktivatoren 4 und 5 über den Behandlungskopf bzw. über das jeweilige Behandlungsrohr 11 Heißluft in die Flaschen 2 eingebracht wird.

Zur Überwachung des Sterilisationsprozesses sind Temperatursensoren 20, 21 und 22 vorgesehen, die jeweils die Temperatur der Flaschen 2 berührungslos erfassen, und zwar unter Berücksichtigung der Infrarot-Strahlung, die von der jeweiligen, durch das Behandlungsmedium erhitzten Flasche 2 ausgeht. Von diesen als Pyrometer oder pyrometerartig ausgebildeten Sensoren ist der Sensor 20 am Auslauf des Sterilisators 3, der Sensor 21 am Auslauf des Aktivators 4 und der Sensor 22 am Auslauf des Aktivators 5 vorgesehen, und zwar jeweils mit dem Rotor 8, 14 bzw. 17 nicht mitdrehend an der Bewegungsbahn der Flaschen 2. Die Signale der Sensoren 20 - 22 werden einer zentralen Steuer- und Überwachungseinheit 23 zugeführt, die u.a. auch die einzelnen Behandlungsstationen des Sterilisators 3 sowie der Aktivatoren 4 und 5 bzw. die dortigen Behandlungsköpfe 10 steuert. Die von den Sensoren 20 - 22 gemessenen Temperaturen T1 (Sensor 20), T2 (Sensor 21) und T3 (Sensor 22) werden in der elektronischen Mess- und Steuereinrichtung mit in einem dortigen Speicher für die jeweilige Flaschenart und/oder Größe abgelegten Soll-Temperaturen verglichen und hieraus Regelsignale zur Ansteuerung der Behandlungsköpfe 10 gebildet. Die Sollwerte für die Temperaturen T1 - T3 betragen beispielsweise:

| | |
|---|---|
| T1_{Soll}: | 45°C |
| T2_{Soll}: | 55°C |
| T3_{Soll}: | 65°C |

Die an den Sensoren 20 - 22 jeweils gemessene Temperatur ist nicht nur abhängig von dem jeweiligen Energieeintrag, der beim Einbringen des Behandlungsmediums in die Flaschen 2 erfolgt, sondern auch von weiteren Parametern. Diese Parameter sind u.a. Flaschengröße und/oder Flaschenart, vor allem aber die Zeitdauer, während der die jeweilige Flasche 2 mit dem jeweiligen Behandlungsmedium behandelt wird. Auch die Abkühlung der Flasche 2 während der Behandlung, d.h. die von der jeweiligen Flasche 2 während der Behandlung an die Umgebung abgegebene Wärmemenge beeinflusst die an den Sensoren 20 - 22 gemessene Temperatur. Die Abkühlung selbst ist, wie ausgeführt, von verschiedenen Einflussgrößen abhängig.

Um den jeweiligen Temperatur-Sollwert für die Temperaturen T1 - T3 zu erreichen, wird über die elektronische Mess- und Steuereinrichtung 23 die Schaltposition SP, an der die jeweilige Behandlung mit dem Behandlungsmedium eingeleitet wird, entsprechend gesteuert, und zwar derart, dass die Behandlungsstrecke, d.h. der Winkelbereich der Drehbewegung des Rotors 8, 14 bzw. 17 zwischen der Schaltposition SP und der Endposition EP, an der die Behandlung mit dem Behandlungsmedium beendet wird und an der bei der dargestellten Ausführungsform auch der jeweilige Sensor 20, 21 bzw. 22 vorgesehen ist, derart geändert wird, dass die ermittelten Temperaturen T1 - T3 dem jeweiligen Temperatursollwert T1_{Soll}, T2_{Soll} bzw. T3_{Soll} möglichst exakt entsprechen. Dies bedeutet im Einzelnen, dass mit steigender Leistung der Anlage 1 die jeweilige Schaltposition SP entgegen der Drehbewegung A, B bzw. C in Richtung Flaschen- bzw. Behältereinlauf 7, 13 bzw. 16 im Sinne einer Vergrößerung der Behandlungsstrecke verlagert wird, wie dies in der Figur 1 mit dem Pfeil SP' angedeutet ist. Umgekehrt erfolgt bei reduzierter Leistung der Anlage 1 eine Verlagerung der Schaltposition SP in Rotordrehrichtung A, B bzw. C im Sinne einer Verkürzung der Behandlungsstrecke, wie dies in der Figur 1 mit dem Pfeil SP" angedeutet ist.

Die Behandlung der Flaschen 2 mit dem jeweiligen Behandlungsmedium wird jeweils an der Endposition EP beendet, d.h. an der Messposition, an der sich auch die Sensoren 20, 21 bzw. 22 befinden, oder aber in Drehrichtung des Rotors 8, 14 bzw. 17 kurz vor Erreichen dieser Messposition.

Bei dieser temperaturgesteuerten Verlängerung oder Verkürzug der Behandlungsstrecke werden zusätzlich zur Leistung der Anlage automatisch auch die anderen vorstehend erwähnten andere Einflussgrößen berücksichtigt. So erfolgt z.B. eine Verlagerung der Schaltposition SP u.a. in Abhängigkeit von der Abkühlung der Flaschen 2 während der Behandlung, von der Umgebungstemperatur, aber auch von der Flaschenart und -größe bzw. von der Wärmekapazität der behandelten Flaschen 2, d.h. bei schwer erwärmbaren Flaschen 2, d.h. bei Flaschen 2 mit hoher Wärmekapazität, erfolgt eine Verlagerung der Schaltposition SP im Sinne des Pfeils SP' und bei leichter erwärmbaren Flaschen 2, d.h. bei Flaschen mit geringer Wärmekapazität eine Verlagerung im Sinne des Pfeils SP".

Solche Flaschen 2, bei deren von dem jeweiligen Sensor 20, 21 bzw. 22 ermittelte Temperatur deutlich von der jeweiligen Solltemperatur abweicht, insbesondere deutlich unter der Solltemperatur liegt, werden beispielsweise als unzureichend behandelte bzw. sterilisierte Flaschen an einer geeigneten Position ausgeschleust, und zwar bevorzugt bereits am Sterilisator 3 bzw. am Aktivator 4 bzw. 5.

Die Erfindung wurde voranstehend an einem Ausführungsbeispiel beschrieben. Es versteht sich, dass zahlreiche Änderungen sowie Abwandlungen möglich sind, ohne dass dadurch der der Erfindung zugrunde liegende Erfindungsgedanke verlassen wird.

So ist es beispielsweise auch möglich, die Endposition EP der jeweiligen Behandlung temperaturabhängig zu verlagern, um so eine temperaturgesteuerte oder -geregelte Verlängerung oder Verkürzung der jeweiligen Behandlungsstrecke zu erreichen.

Weiterhin besteht die Möglichkeit, die Verlängerung oder Verkürzung der Behandlungsstrecke, d.h. die Verlagerung der Schalt- und/oder Endposition SP bzw. EP in Abhängigkeit der jeweiligen aktuellen Maschinenleistung der Anlage 1 zu steuern oder zu regeln.

Weiterhin besteht die Möglichkeit, die Endposition EP der jeweiligen Behandlung oder Behandlungsstrecke in Abhängigkeit von definierbaren Betriebsparametern einzustellen und die Schaltposition PS temperatur- und/oder leistungsgesteuert zu verlagern.

Bei einer Veränderung oder Einstellung der Endposition EP erfolgt bevorzugt auch ein Verschieben des jeweiligen Messpunktes für die Temperaturmessung. Hierfür wird z.B. der Temperatursensor mechanisch auf einer Kreisbahn um die Achse des betreffenden Rotors 8, 14 bzw. 17 verschoben oder aber die Verlagerung des Messpunktes erfolgt dadurch, dass an der Bewegungsbahn der Behandlungsstationen mehrere Temperatursensoren aufeinander folgend vorgesehen sind und dass in Abhängigkeit von der Veränderung oder Einstellung der Endposition EP nur das Messsignal des dieser Endposition entsprechenden Temperatursensors berücksichtigt wird, d.h. beispielsweise auf den dieser Endposition entsprechenden Temperatursensor umgeschaltet wird.

Die verschiedenen Methoden der Steuerung und/oder Regelung sowie Überwachung können auch kombiniert zur Anwendung kommen.

Weiterhin besteht die Möglichkeit, am Flascheneinlauf der Anlage 1 einen weiteren Temperatursensor 24 vorzusehen, mit dem die Eingangstemperatur T0 der Flaschen 2 erfasst wird, wobei dann diese Eingangstemperatur T0 als weiterer Parameter bei der Regelung und/oder Überwachung verwendet wird, beispielsweise zur Bildung entsprechender Solltemperaturen usw.

Vorstehend wurde davon ausgegangen, dass die Anlage 1 zur Sterilisation von Flaschen 2 dient, die im Bereich ihrer Flaschenmündung 2.1 dem Flansch 2.2 aufweisen. Selbstverständlich kann die Anlage 1 bei entsprechender Ausbildung der Flaschen- bzw. Behälterträger 9 auch für andere Flaschenarten Verwendung finden, sowie insbesondere auch für Behälter unterschiedlichster Art ganz allgemein.

### Bezugszeichenliste

- 1: Anlage
- 2: Flasche
- 2.1: Flaschenmündung
- 2.2: Flaschenflansch
- 3: Sterilisator
- 4, 5: Aktivator
- 6: Transporteur
- 7: Einlaufstern
- 8: Rotor
- 9: Flaschen- oder Behälterträger
- 10: Behandlungskopf
- 11: Behandlungsrohr
- 12, 13: Transportstern
- 14: Rotor
- 15, 16: Transportstern
- 17: Rotor
- 18: Auslaufstern
- 19: Transporteur
- 20, 21, 22, 22': Temperatursensor
- 23: elektronische Mess- und Steuereinrichtung
- 24: Temperatursensor

- SP: Schaltposition
- SP', SP": Verlagerung der Schaltposition
- A, B, C: Rotordrehrichtung
- EP: Endposition

## Patentansprüche

1. Verfahren zum Sterilisieren von Flaschen oder dergleichen Behälter (2) durch Einbringen wenigstens eines erhitzten Behandlungsmediums in die Behälter (2), die während der Behandlung mit wenigstens einem Transporteur (8, 14, 17) auf wenigstens einer Behandlungstrecke bewegt werden, die zwischen einer Startposition (SP) und einer Endposition (EP) für das Einleiten und Beenden der Behandlung gebildet ist, wobei die Behältertemperatur nach der Behandlung mit wenigstens einem Temperatursensor (20, 21, 22, 22') erfasst wird, **dadurch gekennzeichnet, dass** die Länge der wenigstens einen Behandlungsstrecke gesteuert oder geregelt so verändert wird, dass die gemessenen Behältertemperatur (T1, T2, T3) zumindest innerhalb eines vorgegebenen Toleranzbereichs einer Solltemperatur (T1_{Soll}, T2_{Soll}, T3_{Soll}) entspricht.

2. Verfahren nach Anspruch 1, **dadurch gekennzeichnet, dass** die Länge der wenigstens einen Behandlungsstrecke temperaturgesteuert oder -geregelt verändert wird, beispielsweise durch einen Vergleich der gemessenen Behältertemperatur als Istwert mit der Solltemperatur (T1_{Soll}, T2_{Soll}, T3_{Soll}).

3. Verfahren nach Anspruch 1 oder 2, **dadurch gekennzeichnet, dass** die Länge der wenigstens einen Behandlungsstrecke in Abhängigkeit von Betriebsparametern, beispielsweise in Abhängigkeit von der Anzahl der je Zeiteinheit behandelten Behälter (2) (Maschinenleistung) und/oder der Behälterart und/oder -größe und/oder de Umgebungstemperatur und/oder -feuchte verändert wird.

4. Verfahren nach einem der vorhergehenden Ansprüche, **dadurch gekennzeichnet, dass** zur Änderung der jeweiligen Behandlungsstrecke deren Schaltposition (SP) verändert wird.

5. Verfahren nach einem der vorhergehenden Ansprüche, **dadurch gekennzeichnet, dass** zur Änderung der jeweiligen Behandlungstrecke deren Endposition (EP) verändert wird.

6. Verfahren nach Anspruch 5, **dadurch gekennzeichnet, dass** bei unter Berücksichtigung von Betriebsparametem eingestellter Endposition (EP) die Startposition temperaturgesteuert verändert wird.

7. Verfahren nach Anspruch 5, **dadurch gekennzeichnet, dass** bei unter Berücksichtigung von Betriebsparametern eingestellter Schaltposition (SP) die Endposition (EP) temperaturgesteuert verändert wird.

8. Verfahren nach einem der vorhergehenden Ansprüche, **dadurch gekennzeichnet, dass** die Behältertemperatur (T1, T2, T3) am Ende der jeweiligen Behandlungsstrecke oder aber unmittelbar nach der Behandlungsstrecke ermittelt wird.

9. Verfahren nach einem der vorhergehenden Ansprüche, **dadurch gekennzeichnet, dass** auf einer Transportstrecke in einer Transportrichtung der Behälter (2) aufeinander folgend wenigstens zwei Behandlungsstrecken gebildet sind, und dass die Länge wenigstens einer Behandlungsstrecke temperaturgesteuert und/oder in Abhängigkeit von Betriebsparametern verändert wird.

10. Verfahren nach Anspruch 9, **dadurch gekennzeichnet, dass** am Ende jeder Behandlungsstrecke oder auf jede Behandlungsstrecke folgend die Temperatur (T1, T2, T3) der Behälter (2) ermittelt wird.

11. Verfahren nach einem der vorhergehenden Ansprüche, **gekennzeichnet durch** die Verwendung von mehreren in Transportrichtung aneinander anschließenden und die Transportstrecke bildenden Transporteuren (8, 14, 17), von denen jeder wenigstens eine Behandlungsstrecke bildet.

12. Verfahren nach einem der vorhergehenden Ansprüche, **dadurch gekennzeichnet, dass** die Behälter (2) auf wenigstens einer Behandlungsstrecke (8) mit einem erhitzten, Wasserstoffperoxyd (H2O2) enthaltenden Sterilisationsmediums, vorzugsweise in Form eines Aerosols, und auf wenigstens einer weiteren in Transportrichtung folgenden Behandlungsstrecke (14, 17) mit Heißluft behandelt werden.

13. Verfahren nach einem der vorhergehenden Ansprüche, **gekennzeichnet durch** die Verwendung wenigstens eines der jeweiligen Behandlungsstrecke zugeordneten Temperatursensors (20, 21, 22, 22') zur vorzugsweise berührungslosen Messung der Temperatur des jeweiligen, an dem Temperatursensor vorbeibewegten Behälters (2).

14. Verfahren nach Anspruch 13, **gekennzeichnet durch** die Verwendung wenigstens eines der wenigstens einen Behandlungsstrecke zugeordneten Pyrometers oder Infrarot-Thermometers als Temperatursensor (20, 21, 22, 22').

15. Verfahren nach einem der vorhergehenden Ansprüche, **dadurch gekennzeichnet, dass** der wenigstens eine Transporteur (8, 14, 17) mehrere Behandlungsstationen zur Aufnahme jeweils eines Behälters (2) aufweist.

16. Verfahren nach einem der vorhergehenden Ansprüche, **dadurch gekennzeichnet, dass** der wenigstens eine Transporteur ein um eine vertikale Achse umlaufend antreibbarer Rotor (8, 14, 17) mit mehreren am Umfang des Rotors ausgebildeten Behandlungsstationen ist, und dass die Behandlungsstrecke jeweils von einem Winkelbereich der Drehbewegung des Rotors (8, 14, 17) gebildet ist.

17. Verfahren nach Anspruch 15 oder 16, **dadurch gekennzeichnet, dass** die Behandlungsstationen jeweils von einem Behälterträger (9) und einem Behandlungskopf (10) gebildet sind, der von einer Mess- und Steuereinrichtung (23) in Abhängigkeit von der gemessenen Behälter-Ist-Temperatur (T1, T2, T3) und dem Temperatur-Sollwert gesteuert wird.

18. Verfahren nach einem der vorhergehenden Ansprüche, **gekennzeichnet durch** die Verwendung einer Transportstrecke mit drei in Transportrichtung der Behälter (2) aneinander anschließenden Behandlungsstrecken, von denen eine erste Behandlungsstrecke zur Behandlung mit dem Wasserstoffperoxyd enthaltenden Sterilisationsmedium und die beiden anschließenden Behandlungsstrecken zur Behandlung mit Heißluft dienen.

19. Verfahren nach Anspruch 18, **dadurch gekennzeichnet, dass** jede Behandlungsstrecke von jeweils einem Transportelement (8, 14, 17) gebildet ist.

20. Vorrichtung zum Sterilisieren von Flaschen oder dergleichen Behälter (2) durch Einbringen wenigstens eines erhitzten Behandlungsmediums in die Behälter (2), umfassend wenigstens einen Transporteur (8, 14, 17), mit dem die Behälter (2) während der Behandlung auf wenigstens einer Behandlungstrecke bewegbar sind, wobei die Behandlungsstrecke zwischen einer Startposition (SP) und einer Endposition (EP) für das Einleiten und Beenden der Behandlung gebildet ist, aufweisend wenigstens einen Temperatursensor (20, 21, 22, 22') zur Erfassung der Behältertemperatur nach der Behandlung, und eine Steuereinrichtung (23), mit der die Länge der wenigstens einen Behandlungsstrecke gesteuert oder geregelt derart veränderbar ist, dass die jeweils gemessenen Behältertemperatur (T1, T2, T3) zumindest innerhalb eines vorgegebenen Toleranzbereichs einer Solltemperatur (T1_{Soll}, T2_{Soll}, T3_{Soll}) entspricht, **dadurch gekennzeichnet, dass** die Steuereinrichtung (23) derart ausgebildet ist, dass sie die Länge der wenigstens einen Behandlungsstrecke temperaturgesteuert oder -geregelt verändert.

21. Vorrichtung nach Anspruch 20, **dadurch gekennzeichnet, dass** die Steuereinrichtung (23) derart ausgebildet ist, dass sie die Länge der wenigstens einen Behandlungsstrecke temperaturgesteuert oder -geregelt durch einen Vergleich der gemessenen Behältertemperatur als Istwert mit der Solltemperatur (T1ₛₒₗₗ, T2_{Soll,} T3_{Soll}). verändert.

22. Vorrichtung nach Anspruch 20 oder 21, **dadurch gekennzeichnet, dass** die Steuereinrichtung (23) derart ausgebildet ist, dass sie die Länge der wenigstens einen Behandlungsstrecke in Abhängigkeit von Betriebsparametern, beispielsweise in Abhängigkeit von der Anzahl der je Zeiteinheit behandelten Behälter (2) (Maschinenleistung) und/oder der Behälterart und/oder -größte und/oder de Umgebungstemperatur und/oder -feuchte verändert.

23. Vorrichtung nach einem der vorhergehenden Ansprüche 20 bis 22, **dadurch gekennzeichnet, dass** die Steuereinrichtung (23) derart ausgebildet ist, dass sie zur Änderung der jeweiligen Behandlungsstrecke deren Schaltposition (SP) verändert.

24. Vorrichtung nach einem der vorhergehenden Ansprüche 20 bis 23, **dadurch gekennzeichnet, dass** die Steuereinrichtung (23) derart ausgebildet ist, dass sie zur Änderung der jeweiligen Behandlungstrecke deren Endposition (EP) verändert.

25. Vorrichtung nach Anspruch 24, **dadurch gekennzeichnet, dass** die Steuereinrichtung (23) derart ausgebildet ist, dass sie bei unter Berücksichtigung von Betriebsparametern eingestellter Endposition (EP) die Startposition temperaturgesteuert verändert.

26. Vorrichtung nach Anspruch 24, **dadurch gekennzeichnet, dass** die Steuereinrichtung (23) derart ausgebildet ist, dass sie bei unter Berücksichtigung von Betriebsparametern eingestellter Schaltposition (SP) die Endposition (EP) temperaturgesteuert verändert.

27. Vorrichtung nach einem der vorhergehenden Ansprüche 20 bis 26, **dadurch gekennzeichnet, dass** der Sensor (20, 21, 22) zur Erfassung der Behältertemperatur (T1, T2, T3) am Ende der jeweiligen Behandlungsstrecke oder aber unmittelbar nach der Behandlungsstrecke angeordnet ist.

28. Vorrichtung nach einem der vorhergehenden Ansprüche 20 bis 27, **dadurch gekennzeichnet, dass** auf einer Transportstrecke in einer Transportrichtung der Behälter (2) aufeinander folgend wenigstens zwei Behandlungsstrecken gebildet sind, und dass die Steuereinrichtung (23) die Länge wenigstens einer Behandlungsstrecke temperaturgesteuert und/oder in Abhängigkeit von Betriebsparametern verändert.

29. Vorrichtung nach Anspruch 28, **dadurch gekennzeichnet, dass** am Ende jeder Behandlungsstrecke oder auf jede Behandlungsstrecke folgend wenigstens ein die Behältertemperatur (T1, T2, T3) erfassender Sensor (20, 21, 22) vorgesehen ist.

30. Vorrichtung nach einem der vorhergehenden Ansprüche 20 bis 29, **gekennzeichnet durch** mehrere in Transportrichtung aneinander anschließende und die Transportstrecke bildende Transporteure (8, 14, 17), von denen jeder wenigstens eine Behandlungsstrecke bildet.

31. Vorrichtung nach einem der vorhergehenden Ansprüche 20 bis 30, **gekennzeichnet durch** wenigstens einen der jeweiligen Behandlungsstrecke zugeordneten Temperatursensor (20, 21, 22, 22') zur vorzugsweise berührungslosen Messung der Temperatur des jeweiligen, an dem Temperatursensor vorbeibewegten Behälters (2).

32. Vorrichtung nach Anspruch 31, **gekennzeichnet durch** wenigstens einen der wenigstens einen Behandlungsstrecke zugeordneten Pyrometers oder Infrarot-Thermometers als Temperatursensor (20, 21, 22, 22').

33. Vorrichtung nach einem der vorhergehenden Ansprüche 20 bis 32, **dadurch gekennzeichnet, dass** der wenigstens eine Transporteur (8, 14, 17) mehrere Behandlungsstationen zur Aufnahme jeweils eines Behälters (2) aufweist.

34. Vorrichtung nach einem der vorhergehenden Ansprüche 20 bis 33, **dadurch gekennzeichnet, dass** der wenigstens eine Transporteur ein um eine vertikale Achse umlaufend antreibbarer Rotor (8, 14, 17) mit mehreren am Umfang des Rotors ausgebildeten Behandlungsstationen ist, und dass die Behandlungsstrecke jeweils von einem Winkelbereich der Drehbewegung des Rotors (8, 14, 17) gebildet ist.

35. Vorrichtung nach Anspruch 33 oder 34, **dadurch gekennzeichnet, dass** die Behandlungsstationen jeweils von einem Behälterträger (9) und einem Behandlungskopf (10) gebildet sind, der von einer Mess- und Steuereinrichtung (23) in Abhängigkeit von der gemessenen Behälter-Ist-Temperatur (T1, T2, T3) und dem Temperatur-Sollwert gesteuert wird.

36. Vorrichtung nach einem der vorhergehenden Ansprüche 20 bis 35, **gekennzeichnet durch** eine Transportstrecke mit drei in Transportrichtung der Behälter (2) aneinander anschließenden Behandlungsstrecken, von denen eine erste Behandlungsstrecke zur Behandlung mit dem Wasserstoffperoxyd enthaltenden Sterilisationsmedium und die beiden anschließenden Behandlungsstrecken zur Behandlung mit Heißluft dienen,

37. Vorrichtung nach Anspruch 36, **dadurch gekennzeichnet, dass** jede Behandlungsstrecke von jeweils einem Transportelement (8, 14, 17) gebildet ist.

## Claims

1. Method for sterilising bottles or similar containers (2) by the introduction of at least one heated treatment medium into the containers (2), which during the treatment are moved by at least one transporter (8, 14, 17) on at least one treatment section which is formed between a start position (SP) and an end position (EP) for the initiating and ending of the treatment, wherein the container temperature after the treatment is detected by means of at least one temperature sensor (20, 21, 22, 22'), **characterised in that** the length of the at least one treatment section is changed in a controlled or regulated manner in such a way that the measured container temperature (T1, T2, T3) corresponds, at least within a specified tolerance range, to a reference temperature (T1_{Ref.}, T2_{Ref.}, T3_{Ref.}).

2. Method according to claim 1, **characterised in that** the length of the at least one treatment section is changed in a temperature-controlled or temperature-regulated manner, for example by a comparison of the measured container temperature as an actual value with the reference temperature (T1_{Ref.}, T2_{Ref.}, T3_{Ref}.).

3. Method according to claim 1 or 2, **characterised in that** the length of the at least one treatment section is changed as a function of operational parameters, for example as a function of the number of containers (2) treated per time unit (machine capacity) and/or the type and/or size of the containers and/or the ambient temperature and/or ambient humidity.

4. Method according to any one of the preceding claims, **characterised in that**, in order to change the respective treatment section, its switching position (SP) is changed.

5. Method according to any one of the preceding claims, **characterised in that**, in order to change the respective treatment section, its end position (EP) is changed.

6. Method according to claim 5, **characterised in that**, with the end position (EP) adjusted by taking account of operating parameters, the start position is changed in a temperature-controlled manner.

7. Method according to claim 5, **characterised in that**, with the switching position (SP) adjusted by taking account of operating parameters, the end position (EP) is changed in a temperature-controlled manner.

8. Method according to any one of the preceding claims, **characterised in that** the container temperature (T1, T2, T3) is determined at the end of the respective treatment section or immediately after the treatment section.

9. Method according to any one of the preceding claims, **characterised in that** at least two treatment sections are formed on a transport section in a transport direction of the containers (2), following one another, and that the length of at least one treatment section is changed in a temperature-controlled manner and/or as a function of operational parameters.

10. Method according to claim 9, **characterised in that**, at the end of each treatment section or following each treatment section, the temperature (T1, T2, T3) of the containers (2) is determined.

11. Method according to any one of the preceding claims, **characterised by** the use of a plurality of transporters (8, 14, 17) connected to one another and forming the transport section, each of which forms at least one treatment section.

12. Method according to any one of the preceding claims, **characterised in that** the containers (2) are treated on at least one treatment section (8) with a heated sterilisation medium containing hydrogen peroxide (H₂O₂), preferably in the form of an aerosol, and on at least one further treatment section (14, 17), following in the transport direction, are treated with hot air.

13. Method according to any one of the preceding claims, **characterised by** the use of at least one of the temperature sensors (20, 21, 22, 22') allocated to the respective treatment section for the preferably contactless measurement of the temperature of the respective container (2) being moved past at the temperature sensor.

14. Method according to claim 13, **characterised by** the use of at least one of the pyrometers or infrared thermometers allocated to the at least one treatment section as a temperature sensor (20, 21, 22, 22').

15. Method according to any one of the preceding claims, **characterised in that** the at least one transporter (8, 14, 17) comprises a plurality of treatment stations for receiving one container (2) in each case.

16. Method according to any one of the preceding claims, **characterised in that** the at least one transporter is a rotor (8, 14, 17) driven such as to rotate about a vertical axis, with a plurality of treatment stations formed at the circumference of the rotor, and that the treatment section is formed in each case by an angle region of the rotational movement of the rotor (8, 14, 17).

17. Method according to claim 15 or 16, **characterised in that** the treatment stations are formed in each case by a container carrier (9) and a treatment head (10), which is controlled by a measurement and control device (23) as a function of the actual temperature measured (T1, T2, T3) of the container and of the temperature reference value.

18. Method according to any one of the preceding claims, **characterised by** the use of a transport section with three treatment sections connected to one another in the transport direction of the containers (2), of which a first treatment section serves to administer the sterilisation medium containing the hydrogen peroxide, and the two following treatment sections serve to administer the treatment with hot air.

19. Method according to claim 18, **characterised in that** each treatment section is formed in each case by a transport element (8, 14, 17).

20. Device for sterilising bottles or similar containers (2) by the introduction of at least one heated treatment medium into the containers (2), comprising at least one transporter (8, 14, 17) with which the containers (2) can be moved during the treatment on at least one treatment section, wherein the treatment section is formed between a start position (SP) and an end position (EP) for the initiating and ending of the treatment, comprising at least one temperature sensor (20, 21, 22, 22') for detecting the container temperature after the treatment, and a control device (23), with which the length of the at least one treatment section can be changed, in a controlled or regulated manner, in such a way that the container temperature (T1, T2, T3) measured in each case corresponds to a reference temperature (T1_{Ref.}, T2_{Ref.}, T3_{Ref.}) at least within a specified tolerance range, **characterised in that** the control device (23) is configured in such a way that it changes the length of the at least one treatment section in a temperature-controlled or temperature-regulated manner.

21. Device according to claim 20, **characterised in that** the controlled device (23) is configured in such a way that it changes the length of the at least one treatment section in a temperature-controlled or temperature-regulated manner by way of a comparison of the measured container temperature as an actual value with the reference temperature (T1_{Ref.}, T2_{Ref.}, T3_{Ref.}).

22. Device according to claim 20 or 21, **characterised in that** the control device (23) is configured in such a way that it changes the length of the at least one treatment section as a function of operating parameters, for example as a function of the number of containers (2) treated per time unit (machine output capacity) and/or of the container type and/or container size and/or the ambient temperature and/or the ambient humidity.

23. Device according to one of the preceding claims 20 to 22, **characterised in that** the control device (23) is configured in such a way that, for the changing of the respective treatment section, it changes its switching position (SP).

24. Device according to one of the preceding claims 20 to 23, **characterised in that** the control device (23) is configured in such a way that, for the changing of the respective treatment section, it changes its end position (EP).

25. Device according to claim 24, **characterised in that** the control device (23) is configured in such a way that it changes the start position in a temperature-controlled manner, taking into account the end position (EP) adjusted by operational parameters.

26. Device according to claim 24, **characterised in that** the control device (23) is configured in such a way that changes the end position (EP) in a temperature-controlled manner, taking into account the switching position (SP) adjusted by operational parameters.

27. Device according to one of the preceding claims 20 to 26, **characterised in that** the sensor (20, 21, 22) for detecting the container temperature (T1, T2, T3) is arranged at the end of the respective treatment section or immediately after the treatment section.

28. Device according to one of the preceding claims 20 to 27, **characterised in that** at least two treatment sections following one another are formed on a transport section in a transport direction of the containers (2), and that the control device (23) changes the length of at least one treatment section in a temperature-controlled manner and/or as a function of operational parameters.

29. Device according to claim 28, **characterised in that** a sensor (20, 21, 22) is provided at the end of each treatment section or following each treatment section, which detects the container temperature (T1, T2, T3).

30. Device according to one of the preceding claims 20 to 29, **characterised by** a plurality of transporters (8, 14, 17), connected to one another in the transport direction and forming the transport section, each of which forms at least one treatment section.

31. Device according to one of the preceding claims 20 to 30, **characterised by** at least one temperature sensor (20, 21, 22, 22') allocated to the respective treatment section for the preferably contactless measurement of the temperature of the respective container (2) moved past at the temperature sensor.

32. Device according to claim 31, **characterised by** at least one pyrometer or infrared thermometer, allocated to the at least one treatment section as a temperature sensor (20, 21, 22, 22').

33. Device according to one of the preceding claims 20 to 32, **characterised in that** the at least one transporter (8, 14, 17) comprises a plurality of treatment stations to receive in each case one container (2).

34. Device according to one of the preceding claims 20 to 33, **characterised in that** the at least one transporter is a rotor (8, 14, 17) driven such as to rotate about a vertical axis, with a plurality of treatment stations formed at the circumference of the rotor, and that the treatment section is formed in each case by an angle region of the rotational movement of the rotor (8, 14, 17)

35. Device according to claim 33 or 34, **characterised in that** the treatment stations are formed in each case by a container carrier (9) and a treatment head (10), which are controlled by a measurement and control device (23) as a function of the actual measured temperature (T1, T2, T3) of the container and of the temperature reference value.

36. Device according to one of the preceding claims 20 to 35, **characterised by** a transport section with three treatment sections connected to one another in the transport direction of the containers (2), of which a first treatment section serves to provide treatment with the sterilisation medium containing hydrogen peroxide and the two following treatment sections serve to provide treatment with hot air.

37. Device according to claim 36, **characterised in that** each treatment section is formed by one transport element (8, 14, 17) in each case.

## Revendications

1. Dispositif servant à stériliser des bouteilles ou des contenants (2) similaires en introduisant au moins un milieu de traitement chauffé dans les contenants (2), qui sont déplacés au cours du traitement à l'aide au moins d'un transporteur (8, 14, 17) sur au moins une ligne de traitement, qui est formée entre une position de départ (SP) et une position finale (EP) pour le début et la fin du traitement, dans lequel la température de contenant est détectée après le traitement à l'aide au moins d'un capteur de température (20, 21, 22, 22'), **caractérisé en ce que** la longueur de l'au moins une ligne de traitement est modifiée par une commande ou par une régulation de telle sorte que la température de contenant (T1, T2, T3) mesurée correspond, au moins dans une plage de tolérance spécifiée, à une température théorique (T1_{théorique}, T2_{théorique,} T3_{théorique}).

2. Procédé selon la revendication 1, **caractérisé en ce que** la longueur de l'au moins une ligne de traitement est modifiée par une commande ou par une régulation de la température, par exemple par une comparaison de la température de contenant mesurée en tant que valeur réelle à la température théorique (T1_{théorique}, T2_{théorique,} T3_{théorique}).

3. Procédé selon la revendication 1 ou 2, **caractérisé en ce que** la longueur de l'au moins une ligne de traitement est modifiée en fonction de paramètres de fonctionnement, par exemple en fonction du nombre des contenants (2) traités par unité de temps (puissance de machine) et/ou du type de contenant et/ou de la taille de contenant et/ou de la température ambiante et/ou de l'humidité ambiante.

4. Procédé selon l'une quelconque des revendications précédentes, **caractérisé en ce que** pour changer la ligne de traitement respective, la position de commutation (SP) de cette dernière est modifiée.

5. Procédé selon l'une quelconque des revendications précédentes, **caractérisé en ce que** pour changer la ligne de traitement respective, la position finale (EP) de cette dernière est modifiée.

6. Procédé selon la revendication 5, **caractérisé en ce que** la position de départ est modifiée par une commande de la température en tenant compte de paramètres de fonctionnement de la position finale (EP) réglée.

7. Procédé selon la revendication 5, **caractérisé en ce que** la position finale (EP) est modifiée par une commande de la température en tenant compte de paramètres de fonctionnement de la position de commutation (SP) réglée.

8. Procédé selon l'une quelconque des revendications précédentes, **caractérisé en ce que** la température de contenant (T1, T2, T3) est déterminée à la fin de la ligne de traitement respective ou toutefois directement après la ligne de traitement.

9. Procédé selon l'une quelconque des revendications précédentes, **caractérisé en ce qu'**au moins deux lignes de traitement se suivant les unes les autres sont formées sur une ligne de transport dans une direction de transport des contenants (2), et que la longueur au moins d'une ligne de traitement est modifiée par une commande de la température et/ou en fonction de paramètres de fonctionnement.

10. Procédé selon la revendication 9, **caractérisé en ce que** la température (T1, T2, T3) des contenants (2) est déterminée à la fin de chaque ligne de traitement ou en suivant chaque ligne de traitement

11. Procédé selon l'une quelconque des revendications précédentes, **caractérisé par** l'utilisation de plusieurs transporteurs (8, 14, 17) se raccordant les uns aux autres dans la direction de transport et formant la ligne de transport, parmi lesquels chaque transporteur forme au moins une ligne de traitement.

12. Procédé selon l'une quelconque des revendications précédentes, **caractérisé en ce que** les contenants (2) sont traités avec un milieu de stérilisation chauffé contenant du peroxyde d'hydrogène (H2O2), de préférence sous la forme d'un aérosol sur au moins une ligne de traitement (8) et à l'air chaud sur au moins une autre ligne de traitement (14, 17) suivant dans la direction de transport.

13. Procédé selon l'une quelconque des revendications précédentes, **caractérisé par** l'utilisation au moins d'un des capteurs de température (20, 21, 22, 22 ') associés à la ligne de traitement respective servant à mesurer de préférence sans contact la température du contenant (2) respectif déplacé le long du capteur de température.

14. Procédé selon la revendication 13, **caractérisé par** l'utilisation en tant que capteur de température (20, 21, 22, 22') au moins d'un pyromètre ou d'un thermomètre infrarouge associé à l'au moins une ligne de traitement.

15. Procédé selon l'une quelconque des revendications précédentes, **caractérisé en ce que** l'au moins un transporteur (8, 14, 17) présente plusieurs postes de traitement servant à recevoir respectivement un contenant (2).

16. Procédé selon l'une quelconque des revendications précédentes, **caractérisé en ce que** l'au moins un transporteur est un rotor (8, 14, 17) pouvant être entraîné en rotation autour d'un axe vertical, pourvu de plusieurs postes de traitement réalisés au niveau de la périphérie du rotor, et que la ligne de traitement est formée respectivement par une zone angulaire du déplacement en rotation du rotor (8, 14, 17).

17. Procédé selon la revendication 15 ou 16, **caractérisé en ce que** les postes de traitement sont formés respectivement par un porte-contenant (9) et par une tête de traitement (10), qui est commandée par un système de mesure et de commande (23) en fonction de la température réelle de contenant (T1, T2, T3) mesurée et de la valeur théorique de la température.

18. Procédé selon l'une quelconque des revendications précédentes, **caractérisé par** l'utilisation d'une ligne de transport avec trois lignes de traitement se raccordant les unes aux autres dans la direction de transport des contenants (2), parmi lesquelles une première ligne de traitement sert au traitement avec le milieu de stérilisation contenant du peroxyde d'hydrogène et les deux lignes de traitement se raccordant servent au traitement à l'air chaud.

19. Procédé selon la revendication 18, **caractérisé en ce que** chaque ligne de traitement est formée par respectivement un élément de transport (8, 14, 17).

20. Dispositif servant à stériliser des bouteilles ou des contenants (2) similaires en introduisant au moins un milieu de traitement chauffé dans les contenants (2), comprenant au moins un transporteur (8, 14, 17), à l'aide duquel les contenants (2) peuvent être déplacés au cours du traitement sur au moins une ligne de traitement, dans lequel la ligne de traitement est formée entre une position de départ (SP) et une position finale (EP) pour le début et la fin du traitement, présentant au moins un capteur de température (20, 21, 22, 22') servant à détecter la température de contenant après le traitement, et un système de commande (23), à l'aide duquel la longueur de l'au moins une ligne de traitement peut être modifiée par une commande ou une régulation de telle manière que la température de contenant (T1, T2, T3) respectivement mesurée correspond au moins dans une plage de tolérance spécifiée à une température théorique (T1_{théorique,} T2_{théorique}, T3_{théorique}), **caractérisé en ce que** le système de commande (23) est réalisé de telle manière qu'il modifie par une commande ou une régulation de la température la longueur de l'au moins une ligne de traitement.

21. Dispositif selon la revendication 20, **caractérisé en ce que** le système de commande (23) est réalisé de telle manière qu'il modifie la longueur de l'au moins une ligne de traitement par une commande ou une régulation de température par une comparaison de la température de contenant mesurée en tant que valeur réelle à la température théorique (T1_{théorique,} T2_{théorique,} T3_{théorique}).

22. Dispositif selon la revendication 20 ou 21, **caractérisé en ce que** le système de commande (23) est réalisé de telle manière qu'il modifie la longueur de l'au moins une ligne de traitement en fonction de paramètres de fonctionnement, par exemple en fonction du nombre des contenants (2) traités par unité temporelle (puissance de machine) et/ou du type de contenant et/ou de la taille de contenant et/ou de la température ambiante et/ou de l'humidité ambiante.

23. Dispositif selon l'une quelconque des revendications précédentes 20 à 22, **caractérisé en ce que** le système de commande (23) est réalisé de telle manière qu'il modifie, afin de changer la ligne de traitement respective, la position de commutation (SP) de cette dernière.

24. Dispositif selon l'une quelconque des revendications précédentes 20 à 23, **caractérisé en ce que** le système de commande (23) est réalisé de telle manière qu'il modifie, afin de changer la ligne de traitement respective, la position finale (EP) de cette dernière.

25. Dispositif selon la revendication 24, **caractérisé en ce que** le système de commande (23) est réalisé de telle manière qu'il modifie par une commande de température la position de départ en tenant compte de paramètres de fonctionnement de la position finale (EP) réglée.

26. Dispositif selon la revendication 24, **caractérisé en ce que** le système de commande (23) est réalisé de telle manière qu'il modifie par une commande de la température la position finale (EP) en tenant compte de paramètres de fonctionnement de la position de commutation (SP) réglée.

27. Dispositif selon l'une quelconque des revendications 20 à 26, **caractérisé en ce que** le capteur (20, 21, 22) servant à détecter la température de contenant (T1, T2, T3) est disposé à la fin de la ligne de traitement respectivement ou toutefois directement après la ligne de traitement.

28. Dispositif selon l'une quelconque des revendications précédentes 20 à 27, **caractérisé en ce qu'**au moins deux lignes de traitement se suivant les unes les autres sont formées sur une ligne de transport dans une direction de transport des contenants (2), et que le système de commande (23) modifie la longueur au moins d'une ligne de traitement par une commande de la température et/ou en fonction de paramètres de fonctionnement.

29. Dispositif selon la revendication 28, **caractérisé en ce qu'**au moins un capteur (20, 21, 22) détectant la température de contenant (T1, T2, T3) est prévu à la fin de chaque ligne de traitement ou en suivant chaque ligne de traitement.

30. Dispositif selon l'une quelconque des revendications précédentes 20 à 29, **caractérisé par** plusieurs transporteurs (8, 14, 17) se raccordant les uns les autres dans la direction de transport et formant la ligne de transport, parmi lesquels chaque transporteur forme au moins une ligne de traitement.

31. Dispositif selon l'une quelconque des revendications précédentes 20 à 30, **caractérisé par** au moins un capteur de température (20, 21, 22, 22') associé à la ligne de traitement respective, servant à mesurer de préférence sans contact la température du contenant (2) respectif déplacé le long du capteur de température.

32. Dispositif selon la revendication 31, **caractérisé par** au moins un pyromètre ou un thermomètre infrarouge associé à l'au moins une ligne de traitement en tant que capteur de température (20, 21, 22, 22').

33. Dispositif selon l'une quelconque des revendications précédentes 20 à 32, **caractérisé en ce que** l'au moins un transporteur (8, 14, 17) présente plusieurs postes de traitement servant à recevoir respectivement un contenant (2).

34. Dispositif selon l'une quelconque des revendications précédentes 20 à 33, **caractérisé en ce que** l'au moins un transporteur est un rotor (8, 14, 17) pouvant être entraîné en rotation autour d'un axe vertical, pourvu de plusieurs postes de traitement réalisés au niveau de la périphérie du rotor, et que la ligne de traitement est formée respectivement par une zone angulaire du déplacement en rotation du rotor (8, 14, 17).

35. Dispositif selon la revendication 33 ou 34, **caractérisé en ce que** les postes de traitement sont formés respectivement par un porte-contenant (9) et une tête de traitement (10), qui est commandée par un système de mesure et de commande (23) en fonction de la température réelle de contenant (T1, T2, T3) mesurée et la valeur théorique de température.

36. Dispositif selon l'une quelconque des revendications précédentes 20 à 35, **caractérisé par** une ligne de transport avec trois lignes de traitement se raccordant les unes les autres dans la direction de transport des contenants (2), parmi lesquelles une première ligne de traitement sert au traitement avec le milieu de stérilisation contenant du peroxyde d'hydrogène et les deux lignes de traitement se raccordant servent au traitement à l'air chaud.

37. Dispositif selon la revendication 36, **caractérisé en ce que** chaque ligne de traitement est formée par respectivement un élément de transport (8, 14, 17).
